# EUROPEAN PATENT SPECIFICATION

(11) **EP 4 463 869 B1**
(45) Date of publication and mention of the grant of the patent: **11.11.2026**
(21) Application number: 23700510.3
(22) Date of filing: 10.01.2023
(51) Int. Cl.: G16C 20/20, G06N 3/02

(54) **METHOD FOR STRUCTURE ELUCIDATION**
VERFAHREN ZUR STRUKTURAUFKLÄRUNG
PROCÉDÉ D'ÉLUCIDATION DE STRUCTURE

(30) Priority: 12.01.2022 EP 22151206
(43) Date of publication of application: 20.11.2024
(73) Proprietor: Boehringer Ingelheim International GmbH, 55216 Ingelheim am Rhein (DE)
(72) Inventor: REDDIG, Tim, 55216 Ingelheim (DE); LEKIC, Vladimir, 55216 Ingelheim (DE); ROTTACH, Florian, 55216 Ingelheim (DE)
(74) Representative: Von Rohr Patentanwälte Partnerschaft mbB
(86) International application number: PCT/EP2023/050395
(87) International publication number: WO 2023/135113

(56) References cited:
- ZHANG JINZHE ET AL: "NMR-TS: de novo molecule identification from NMR spectra", vol. 21, no. 1, 31 January 2020 (2020-01-31), pages 552 - 561, XP055934535, ISSN: 1468-6996, Retrieved from the Internet <URL:https://www.tandfonline.com/doi/pdf/10.1080/14686996.2020.1793382> DOI: 10.1080/14686996.2020.1793382
- NICOLA DE CAO ET AL: "MolGAN: An implicit generative model for small molecular graphs", ARXIV.ORG, CORNELL UNIVERSITY LIBRARY, 201 OLIN LIBRARY CORNELL UNIVERSITY ITHACA, NY 14853, 30 May 2018 (2018-05-30), XP080883898
- LAI-WAN CHAN ET AL: "The prediction of carbon-13 NMR chemical shifts using ensembles of networks", NEURAL NETWORKS PROCEEDINGS, 1998. IEEE WORLD CONGRESS ON COMPUTATIONA L INTELLIGENCE. THE 1998 IEEE INTERNATIONAL JOINT CONFERENCE ON ANCHORAGE, AK, USA 4-9 MAY 1998, NEW YORK, NY, USA,IEEE, US, vol. 1, 4 May 1998 (1998-05-04), pages 96 - 100, XP010286529, ISBN: 978-0-7803-4859-2, DOI: 10.1109/IJCNN.1998.682243
- AIRES-DE-SOUSA JOÃO ET AL: "Prediction of 1 H NMR Chemical Shifts Using Neural Networks", ANALYTICAL CHEMISTRY, vol. 74, no. 1, 1 December 2001 (2001-12-01), US, pages 80 - 90, XP055934906, ISSN: 0003-2700, DOI: 10.1021/ac010737m
- HUANG ZHAORUI ET AL: "A framework for automated structure elucidation from routine NMR spectra", vol. 12, no. 46, 1 December 2021 (2021-12-01), United Kingdom, pages 15329 - 15338, XP055934539, ISSN: 2041-6520, Retrieved from the Internet <URL:https://pubs.rsc.org/en/content/articlepdf/2021/sc/d1sc04105c> DOI: 10.1039/D1SC04105C

## Description

The present invention relates to a method for structure elucidation of the structure or molecular structure of an unknown chemical compound from a measured spectrum of a sample, as well as to a data processing apparatus, a computer program product and a computer-readable storage medium

In chemistry and pharmacy, it is often important to analyze the chemical composition of a sample or to analyze the chemical compounds contained in a sample. For example, the sample may be or have a medicament that is analyzed regarding potential impurities. In another example, the sample may be or have a newly synthesized chemical compound that has to be verified. For analyzing the sample, several methods are available, for example nuclear magnetic resonance (hereinafter abbreviated as NMR) spectroscopy, mass spectrometry, infrared spectroscopy, Raman spectroscopy, and X-ray crystallography, to name just a few.

In organic chemistry and pharmacy, NMR spectroscopy is one of the most-used methods to identify chemical compounds in a sample. While this method has a number of advantages, it is not possible to infer the geometric or molecular structure of the measured chemical compound directly from the NMR spectrum.

Experimental NMR spectra typically provide a thorough description of the hydrocarbon framework of organic molecules, but a complete one-to-one assignment of observed features in the spectrum (in particular chemical shifts) to individual nuclei of the sample under investigation can be very challenging if the measured spectrum does not match with a known spectrum of a known chemical compound.

For example, a typical task in the production of a drug or medicament or other chemical product is to verify that the desired drug or medicament or chemical product has in fact being synthesized or produced and/or to check if impurities are present in the sample and, in the affirmative, to determine the impurities. For this, the measured spectra, in particular NMR spectra, of the sample are compared with the expected spectra of the chemical compounds contained or expected in the sample.

It is quite easy to verify that the desired chemical compounds are present in the sample because this is the case if the measured spectra match the expected known spectra of the chemical compounds present in the sample. However, there may be unknown chemical compounds in the sample, for example when impurities are present or when the synthesis has not resulted in the desired chemical compound. In this case, it can be very difficult to assign the measured spectrum to a chemical compound.

In the context of the present disclosure, the term "structure elucidation" denotes the process or method of determining the geometric or molecular structure of a chemical compound contained in a sample from a measured spectrum of the sample.

The task of structure elucidation is to find the molecular structure that best matches the measured spectrum, in particular NMR spectrum, of the sample. However, structure elucidation is a very tedious and time-consuming process and usually requires an expert having a lot of experience and expertise.

Thus, it is desirable to have a fully automated method for structure elucidation.

Zhang et al (2020), "NMR-TS: de novo molecule identification from NMR spectra", Science and Technology of Advanced Materials, 21(1), 552-56 relates to molecule identification from NMR spectra. Structures of candidate chemical compounds are generated with the molecule generator "ChemTS", which uses a recurrent neural network (RNN).

De Cao et al (2018), "MolGAN: An implicit generative model for small molecular graphs", arXiv:1805.11973 relates to generation of molecular structures using neural networks.

Chan et al (1998), "The prediction of carbon-13 NMR chemical shifts using ensembles of networks", 1998 IEEE International Joint Conference on Neural Networks Proceedings. IEEE World Congress on Computational Intelligence (Cat. No.98CH36227), Volume 1, 96-100 relates to the generation of NMR spectra using four multi-layer perceptrons.

Aires-de-Sousa et al (2002), "Prediction of 1H NMR Chemical Shifts Using Neural Networks", Analytical Chemistry, Volume 74, Issue 1, pages 80-90 relates to the generation of NMR spectra using counterpropagation neural networks.

The object of the present invention is to provide a method for structure elucidation which is fully automated, quick and/or reliable.

The above object is solved by the method according to claim 1, the data processing apparatus according to claim 12, the computer program product according to claim 13 or the computer-readable storage medium according to claim 14. Advantageous developments are subject of the dependent claims.

In particular, the present invention relates to a method for structure elucidation of the structure of an unknown chemical compound from a measured spectrum of a sample. The term "structure" denotes in particular the molecular structure of a chemical compound or its molecules and is defined further below.

In the method according to the invention, structures of candidate chemical compounds are generated and from these generated structures, predicted spectra are generated. The predicted spectra are compared with the measured spectrum. One of the predicted spectra is selected, in particular based on the comparison, and the structure corresponding to the selected predicted spectrum is determined as the structure of the unknown chemical compound.

According to a first aspect, a first machine learning model generates the structures of chemical compounds and a second machine learning model generates the predicted spectra from the structures generated by the first machine learning model. By this, structure elucidation is made very fast, reliable and efficient. In particular, the method for structure elucidation can be fully automated or at least automated in large parts and/or consulting an expert for structure elucidation can be avoided.

According to another aspect which can also be implemented independently, a machine learning model, hereinafter referred to as first machine learning model, generates structures of candidate chemical compounds, wherein the first machine learning model is trained for generation of realistic structures from a molecular and/or empirical formula. This is conducive to a fast, reliable and efficient structure elucidation. In particular, at least a part of the structure elucidation can be automated, namely providing a possible or candidate chemical compound or, in other words, identifying candidates of the unknown chemical compound.

In all aspects and embodiments of the invention, a machine learning model, hereinafter referred to as second machine learning model, generates the predicted spectra from the molecular structures, the second machine learning model having a residual neural network. This is conducive to making the structure elucidation fast, reliable and efficient. In particular, the structure elucidation can be automated at least in parts. Particularly, spectra of the candidate chemical compounds or candidates can be generated automatically.

Advantageously, the first machine learning model has and/or uses one or more artificial neural networks, preferably one or more graph neural networks, in particular a pair of generative adversarial networks. The use of a graph neural networks or generative adversarial networks has proven to be particularly suitable and efficient for generating structures.

The first machine learning model is preferably trained with a first training dataset. Preferably, the first machine learning model is trained for the generation of realistic structures. In particular, the first machine learning model is trained for the generation of realistic structures from molecular and/or empirical formulas. The first machine learning model preferably comprises the first training dataset and/or the first training dataset preferably forms a part of the first machine learning model. By training the first machine learning model with the first training dataset, it can be achieved that the first machine learning model only generates structures that are chemically and/or physically possible. In particular, if the first training dataset is large and diverse enough, the first machine learning model can preferably learn to generate novel structures, i.e. structures that are not contained in the first training dataset.

The first training dataset preferably comprises structures of a plurality of real molecules. In this way, the first machine learning model can be efficiently trained to generate only realistic structures. The generation of structures that are chemically and/or physically impossible is preferably avoided or at least significantly reduced.

It is advantageous if the first machine learning has a generator and preferably a discriminator and/or to train the first machine learning model using a generator and preferably a discriminator. The generator is in particular an object that generates and/or is trained to generate structures from molecular and/or empirical formulas. The discriminator is in particular an object that differentiates and/or is trained to differentiate between real structures, in particular from the first training dataset, and other or artificial structures, in particular structures that have been generated by the generator. In particular during training the generator is fed with the results and/or decisions of the discriminator and the discriminator is fed with both the structures, in particular from the first dataset, and the structures generated by the generator. In this way, the generator and discriminator are mutually trained.

In this way, on the one hand, the generator can be trained to generate only structures that are realistic and/or physically and/or chemically possible. On the other hand, the discriminator is in this way trained to learn how a "real" molecule looks and to differentiate real molecules from artificial molecules or structures generated by the generator.

In particular, the generator and the discriminator are mutually trained. This leads to a generator that, after training, produces realistic and/or chemically and/or physically possible molecules or structures.

The discriminator is preferably used only in the training and/or not used in the actual structure elucidation and/or not used in the application phase. Here, the term "actual structure elucidation" in particular means the step of generating molecules after training of the first machine learning model is completed.

The first machine learning model, in particular the generator, preferably generates and/or is trained to generate several structures from a given molecular and/or empirical formula. By this, the success rate of the method for structure elucidation can be improved.

It is preferred that the first machine learning model, in particular the generator, has and/or uses a random noise generator and/or random variables. By the random noise generator or random variables, it can in particular be achieved or ensured that several different structures are generated from one given molecular and/or empirical formula. In particular, diversity in generated structures is ensured through the usage of the random noise generator or random variables. This is conducive to a high success rate in structure elucidation.

The discriminator is preferably trained to differentiate between real structures, in particular from the first training dataset, and structures generated by the generator. By the discriminator, the generator can be efficiently trained for the generation of the realistic and/or chemically and/or physically possible structures.

The second machine learning model a residual neural network. The use of an artificial neural network, in particular a graph attention network and/or residual neural network, has proven to be advantageous in the generation of predicted spectra, in particular NMR spectra, from a given structure. This leads to a fast and/or reliable generation of predicted spectra and in particular to highly accurate predicted spectra. In particular, the use of a graph neural network and/or residual neural network allows for a decrease in the number of needed external training samples and allows to calculate spectra, in particular NMR spectra, of molecules of unlimited size. Further, the generation of predicted spectra can be parallelized. Surprisingly, due to the use of the graph neural network, graph attention network and/or residual neural network, the present method for structure elucidation and/or the second machine learning model is able to predict the different chemical shifts of diastereotopic protons in NMR spectra.

The second machine learning model is preferably trained with a second training dataset. The second training dataset is preferably different from the first dataset and/or contains data having a different structure and/or different information than the data from the first training dataset.

In particular, the second training dataset contains structures labeled with related spectrum features. In other words, for every structure in the second training dataset, the second training dataset (additionally) contains information about the features that the structure will give rise to when its spectrum is measured. Preferably, the spectrum is an NMR spectrum and/or the spectrum features are chemical shifts, in particular chemical shifts of ¹H and/or ¹³C.

It is preferred that, in the generation of predicted spectra, an expected or mean value and a corresponding measure of dispersion, in particular a standard deviation, are calculated for each feature of the spectrum, in particular for each chemical shift of the spectrum. By this, it is even possible to predict the different spectrum features, in particular chemical shifts, of diastereotopic protons.

Preferably, the measured spectrum is an NMR spectrum. Particularly, the spectrum, in particular the NMR spectrum, of the sample is measured. However, measuring the spectrum is not an essential feature of the method. It is also possible to perform the method without the explicit step of measuring the sample, for example when the sample has already been measured before and/or independently from the method and/or a when measured spectrum is present as a dataset or the like. Thus, the step of measuring the spectrum of the sample can be separate from the inventive method and is preferably a step that precedes it.

The molecular and/or empirical formula is preferably determined by measuring a mass spectrum of the sample.

The method according to the invention is preferably a computer-implemented method. In this way, the method can be at least partially or fully automated.

According to another aspect, the present invention relates to a data processing apparatus comprising means for carrying out the method.

According to another aspect, the present invention relates to a computer program product comprising instructions which, when the program is executed by a computer, cause the computer to carry out the method.

According to another aspect, the present invention relates to a computer-readable storage medium comprising instructions which, when executed by a computer, cause the computer to carry out the method.

A "molecular structure" in the sense of the present disclosure is preferably the geometrical structure of a molecule, in particular the geometrical and/or three-dimensional arrangement of the atoms of the molecule.

An "empirical formula" of a chemical compound in the sense of the present disclosure is preferably the simplest whole number ratio of atoms present in the chemical compound. The empirical formula makes no mention of the arrangement or number of atoms. In particular, the total number of atoms of a given chemical compound cannot be inferred from its empirical formula. A simple example of this concept is that the empirical formula of sulfur monoxide (SO) would simply be SO as is the empirical formula of disulfur dioxide (S₂O₂). Thus, sulfur monoxide and disulfur dioxide have the same empirical formula (SO), while sulfur monoxide has only one sulfur atom and one oxygen atom, whereas disulfur dioxide has two sulfur atoms and two oxygen atoms.

A "molecular formula" in the sense of the present disclosure preferably indicates the numbers of each type of atom in a molecule of a chemical compound. The molecular formula is the same as the empirical formula for molecules that only have one atom of the particular type. Otherwise, the molecular formula may have larger numbers. In the above example, the molecular formula of sulfur monoxide is SO and, thus, the same as the empirical formula. For disulfur dioxide, the molecular formula is S₂O₂ and, thus, different from the empirical formula SO.

Both the empirical formula and the molecular formula do not give any information about the molecular or geometric structure of the molecule.

A "chemical compound" in the sense of the present disclosure is preferably a chemical substance composed of a plurality of identical molecules.

A "structure" of a chemical compound or its molecules in the sense of the present invention is preferably the molecular structure of the chemical compound or its molecules, or, in other words, the two- and/or three-dimensional arrangement of the individual atoms of the molecule. Thus, the term "structure" denotes in particular the arrangement of the atoms or nuclei of the chemical compound or its molecules. The structure or molecular structure can also be present as or represented by a formula representing the geometrical/molecular structure, in particular a structural formula or skeletal formula or the like. For example, the structural formula of ethanol (C₂H₆O) reads:

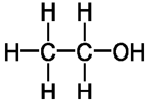

A "candidate chemical compound" in the sense of the present disclosure is preferably a chemical compound that is a candidate of being the unknown chemical compound in the sample of which the structure is unknown and/or to be elucidated. In other words, a candidate chemical compound is a chemical compound that might be or probably is the unknown chemical compound. In particular, the empirical and/or molecular formula of the candidate chemical compound is the same as the empirical and/or molecular formula of the unknown chemical compound, which in particular has or can be measured, for example by performing a mass spectrometry of the sample and/or unknown chemical compound.

A "measured spectrum" in the sense of the present disclosure is preferably the result of a measurement of the sample by a spectroscopic method, for example NMR spectroscopy, mass spectrometry , infrared spectroscopy, Raman spectroscopy, X-ray crystallography, or the like. Particularly preferably, however, the spectrum is an NMR spectrum. The measured spectrum is preferably present as and/or represented by a set of data or data points, in particular digital data. The term "measured spectrum" serves in particular to differentiate the measured spectrum from a predicted spectrum, which is explained below.

A "predicted spectrum" in the sense of the present invention is a spectrum that has not been actually measured and is generated by the second machine learning model. The predicted spectrum is preferably present as and/or represented by a set of data or data points, in particular digital data. In particular, the predicted spectrum has the same data type and/or data structure as the measured spectrum.

A "nuclear magnetic resonance spectrum", hereinafter shortly referred to as "NMR spectrum", in the sense of the present disclosure is preferably a spectrum measured by NMR spectroscopy or a predicted spectrum that has the same datatype or data structure as a measured NMR spectrum. An NMR spectrum in particular has or consists of several features. These features are in particular peaks in the spectrum. In the context of NMR, the features and/or peaks of the spectrum are in particular denoted as "chemical shifts". A chemical shift is the resonant frequency of a nucleus relative to a standard in a magnetic field. The chemical shift is preferably expressed in ppm.

A "machine learning model" in the sense of the present disclosure preferably comprises and/or uses a machine learning algorithm and one or more (training) datasets. In other words, a machine learning model is preferably the output of a machine learning algorithm run on one or more (training) datasets. In particular, a machine learning model represents what was learned by a machine learning algorithm. An algorithm is in particular a procedure that is run on one or more (training) datasets to create the machine learning model. The algorithm may in particular be an artificial neural network, particular preferably a graph neural network.

A "training dataset" in the sense of the present disclosure is preferably a set of data that is used to train the machine learning model and/or machine learning algorithm.

An "artificial neural network" in the sense of the present disclosure is preferably a computational learning system that uses a network of functions to understand and translate a data input of one form into a desired output, usually in another form. A neural network is composed of at least two, preferably three or more layers. In particular, an artificial neural network has an input layer, an output layer and one or more hidden layers, i.e. layers between the input layer and the output layer. Each layer has one or more units called "neurons". The concept of artificial neural networks is inspired by the brain and the way a brain learns.

A "graph neural network" in the sense of the present disclosure is preferably an artificial neural network that has and/or utilizes and/or can be directly applied to graphs.

A "graph" in the sense of the present disclosure is preferably a data structure consisting of nodes and edges. In the context of artificial neural networks, the nodes of a graph can represent entities, such as different people, and the edges of a graph can represent relations or links between the nodes, such as personal relationships between the people. In another example, the nodes may represent nuclei of a molecule and the edges may represent chemical bonds between the nuclei. In an implementation of a machine learning model, a graph may be represented as a matrix, in particular an adjacency matrix.

A "feature vector" in the sense of the present disclosure is preferably an array or vector assigned to a node. A feature vector has one or more elements that contain information about the node.

An "embedding" in the sense of the present disclosure is preferably a low-dimensional space or vector into which high-dimensional vectors can be translated. An embedding can in particular be learned. In particular, a node embedding is a, particularly low-dimensional, vector representation of nodes in a graph.

The above-mentioned aspects and features of the present invention and the aspects and features of the present invention that will become apparent from the claims and the following description can in principle be implemented independently from one another, but also in any combination or order.

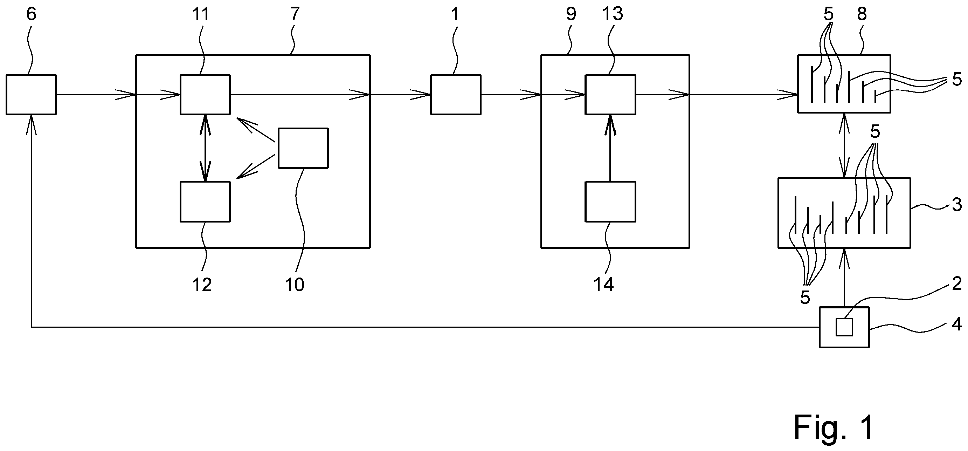

Further aspects, advantageous, features and properties of the present invention will become apparent from the claims and the following description of a preferred embodiment with reference to the drawings, in which Fig. 1 shows a schematic depiction of the inventive method.

The method for structure elucidation according to the invention is schematically depicted in Fig. 1. The method is in particular a method for structure elucidation of the structure 1, in particular geometric and/or molecular structure 1, of an unknown chemical compound 2 from a measured spectrum 3 of a sample 4.

The method is preferably used for finding and/or determining a contamination of the sample 4. In this case, the unknown chemical compound 2 is a contamination. The sample 4 in this case is preferably a medicament or a drug.

The method is preferably a computer-implemented method.

First, a brief overview over the method is given and then, further details of different method steps are explicated.

### Overview

First, a spectrum 3 of a sample 4 is preferably measured. This spectrum 3 is in the following denoted as measured spectrum 3.

While the presence of a measured spectrum 3 of the sample 4 is a necessary requirement for performing the method, the actual step of measuring the spectrum 3 is not an essential feature of the present method. In particular, the step of measuring the spectrum 3 of the sample 4 can take place separately from and in particular precede the present method.

The measured spectrum 3 is particularly preferably an NMR spectrum of the sample but can in principle also be a spectrum 3 measured by another spectroscopic method than NMR.

The sample 4 preferably is or comprises a medicament or drug but can be any sample 4 that is analyzable by a spectroscopic method, in particular NMR. The sample 4 preferably comprises at least one active ingredient.

The sample 4 preferably comprises an unknown chemical compound 2. The unknown chemical compound 2 gives rise to features in the measured spectrum 3 that cannot be assigned to known or expected chemical compounds (assumably) contained in the sample 4. Thus, the presence of features in the measured spectrum 3 that cannot be assigned to known and/or expected chemical compounds is a hint that an unknown chemical compound 2 is contained in the sample 4. This may be due to the presence of impurities in the sample 4 and/or due to a synthesis that has not or not only resulted in the expected or desired chemical compounds to be synthesized.

The sample 4 may be a sample in which the unknown unknown chemical compound 2 has been isolated and/or concentrated.

The measured spectrum 3 preferably comprises one or more spectrum features 5.

The spectrum features 5 are preferably peaks in the measured spectrum 3. Particularly, in the case of NMR spectra, the spectrum features 5 are chemical shifts.

Preferably, a molecular and/or empirical formula 6 of the unknown chemical compound 2 is determined. This can for example be done by performing a mass spectrometry of the sample 4 and/or the, in particular isolated and/or concentrated, unknown chemical compound 2. The step of determining the molecular and/or empirical formula 6 of the unknown chemical compound 2 is not an essential feature of the inventive method and preferably precedes it.

For structure elucidation, structures 1 of candidate chemical compounds are preferably generated. In particular, several different structures 1 of candidate chemical compounds are generated from one or the same molecular and/or empirical formula 6. This is preferably done by a machine learning model 7, hereinafter in particular referred to as first machine learning model 7.

From the generated structures 1, predicted spectra 8 are preferably generated. In particular, for each generated structure 1, exactly one predicted spectrum 8 is generated. This is preferably done by a machine learning model 9, hereinafter in particular referred to as second machine learning model 9.

The terms "first" and "second" machine learning model do not imply an order of the machine learning models 7, 9 but serve to differentiate between the machine learning models. Thus, the prefixes "first" and "second" can also be left out. Therefore, the first machine learning model 7 can also be denoted as machine learning model 7 and the second machine learning model 9 can also be denoted as machine learning model 9.

In particular, it is also possible that the method comprises/uses only one machine learning model, i.e. either only the (first) machine learning model 7 or only the (second) machine learning model 9. However, the method according to the invention always uses the (second) machine learning model 9 having a residual neural network.

The first machine learning model 7 and/or the second machine learning model 9 preferably (each) have an algorithm, in particular a machine learning algorithm, and/or a training dataset. Preferably, the first machine learning 7 has a different algorithm than the second machine learning model 9 and/or the first machine learning model 7 has a different training dataset than the second machine learning model 9. Preferably, the algorithm of the first machine learning model 7 and/or the second machine learning model 9 is an artificial neural network, in particular a graph neural network.

The predicted spectra 8 are preferably of the same kind as the measured spectrum 3 and/or have the same data structure as the measured spectrum 3. For example, if the measured spectrum 3 is an NMR spectrum, the predicted spectra 8 are also NMR spectra. In particular, the (only) difference between the predicted spectra 8 and the measured spectrum 3 is that the measured spectrum 3 is or has been actually measured with the sample 4, whereas the predicted spectra 8 have been artificially generated and/or computed, in particular by the second machine learning model 9. The different terms "measured spectrum 3" and "predicted spectrum 8" predominantly only serve two differentiate between the spectrum 3 that has been measured with the sample and the spectra 8 that have been artificially generated and/or computed.

The structures 1 are in particular molecular structures or, in other words, geometric, two-dimensional and/or three-dimensional structures 1 of molecules. The structures 1 are preferably present as or represented by data defining the relative positions of atoms or nuclei in the molecule but may also be present as or represented by structural formulas, skeletal formulas, or other suitable data and/or formulas.

The structures 1 are preferably computed or generated starting from the molecular and/or empirical formula(s) 6.

After generating the predicted spectra 8, the predicted spectra 8 are preferably compared with the measured spectrum 3. This is preferably done automatically and/or computer-implemented.

Then, one of the predicted spectra 8 is preferably selected. This is preferably done on the basis of the comparison with the predicted spectra 8 with the measured spectrum 3. In particular, the predicted spectrum 8 that best matches the measured spectrum 3 is selected.

The selection of a predicted spectrum 8 is in particular the decision which of the predicted spectra 8 best matches the measured spectrum 3. The predicted spectra 8 which are not selected are preferably discarded/rejected.

Finally, the structure 1 corresponding to the selected predicted spectrum 8, i.e. in particular the structure 1 from which the selected predicted spectrum 8 has been generated, is preferably determined as the structure 1 of the unknown chemical compound 2. This is preferably done automatically and/or computer-implemented.

In particular, all steps of the method are performed automatically and/or computer-implemented and/or the method is a fully automated and/or computer-implemented method.

It is a preferred aspect of the present invention that a first machine learning model 7 is used that generates the structures 1 of candidate chemical compounds and a second machine learning model 9 is used that generates predicted spectra 8 from the structures 1 generated by the first machine learning model 7. In other words, it is preferred that the method for structure elucidation utilizes two different machine learning models 7, 9 that have different learning algorithms and/or that are trained differently, that are trained with different training datasets and/or that are trained for different tasks. In particular, the task of generating structures 1 or candidate chemical compounds is separated from the task of generating predicted spectra 8. By this, an effective and/or efficient training is possible and both the generation of possible structures 1 of (candidate) chemical compounds and the generation of predicted spectra 8 can be made more efficient, quick and/or reliable.

By the general idea of the above aspect, namely performing a structure elucidation by first generating structures 1 of candidate chemical compounds and then generating predicted spectra 8 of these structures 1, the fundamental problem of structure elucidation, namely the direct inference to a molecular structure from a measured a spectrum 3 is circumvented and, in this way, structure elucidation can be made much quicker and more efficient.

### First machine learning model

According to a preferred aspect, which can also be implemented independently, generating the structures 1 of candidate chemical compounds is done by a machine learning model, in particular the first machine learning model 7.

The first machine learning model 7 is preferably trained for generating realistic structures 1. A realistic structure in this sense is in particular a structure that is physically and/or chemically possible. The structures 1 are preferably generated from a molecular and/or empirical formula 6.

In particular, the first machine learning model 7 is trained before using the first machine learning model 7 for actual structure elucidation. In other words, the method according to the present invention preferably involves a training or training phase of the first machine learning model 7 and an application phase of the first machine learning model 7.

In the training or training phase, the first machine learning model 7 preferably learns how realistic structures 1 are generated, in particular starting from a molecular and/or empirical formula 6. After training or when the training phase is finished, the first machine learning model 7 is or can be used for generating structures 1 of candidate chemical compounds, in particular in order to elucidate the structure 1 of an unknown chemical compound 2 from a measured spectrum 3 of a sample 4.

The application or application phase is preferably the phase after finishing the training phase or, in other words, the phase in which the first machine learning model 7 is used for generating structures 1 of candidate chemical compounds and/or for actual structure elucidation.

The first machine learning model 7 is preferably trained with a training dataset 10, hereinafter referred to as first training dataset 10. Preferably, the first machine learning model 7 comprises the first training dataset 10 and/or the first training dataset 10 forms a part or component of the first machine learning model 7.

The first machine learning model 7 is particularly trained for generating realistic structures 1. In other words, the object of the training of the first machine learning model 7 is to achieve or ensure that the structures 1 generated by the first machine learning model 7 are physically and/or chemically possible. This is in particular achieved by choosing an appropriate first training dataset 10.

The first training dataset 10 preferably comprises structures 1 of a plurality of real molecules or chemical compounds. The structures 1 are preferably present as and/or represented by digital data. The structures 1 of real molecules or chemical compounds may, for example, be present or provided as data defining or containing the relative geometrical positions of atoms or nuclei of the molecule or chemical compound and/or as a formula representing the structure 1, such as a structural formula or skeletal formula.

The first training dataset 10 is preferably a database or taken from a database. A preferred example for the first training dataset 10 and/or database is the ZINC database, which is available under https://zinc.docking.org. The ZINC database is described in more detail in J. Chem. Inf. Model. 2012, 52, 7, 1757-1768, published on May 15, 2012.

The first training dataset 10 preferably comprises molecular and/or empirical formulas 6, in particular in addition to the structures 1 of real molecules or chemical compounds. Particularly preferably, for every molecule or chemical compound in the first training dataset 10, the first training dataset 10 comprises the structure 1 and the molecular and/or empirical formula 6 of the respective molecule or chemical compound.

The first machine learning model 7 is in particular trained for generating structures 1 from a molecular and/or empirical formula 6. In other words, it is preferred that the molecular and/or empirical formula 6 is used as or constitutes an input for the first machine learning model 7. Then, the machine learning model 7 generates one or more structures 1 from the input molecular and/or empirical formula 6. The generated structures 1 preferably constitute an output of the first machine learning model 7. This is also schematically depicted in Fig. 1.

The structures 1 generated by the first machine learning model 7 may, for example, be present or provided as data defining or containing their relative geometrical positions of atoms or nuclei of the molecule or chemical compound and/or as a formula representing the structure 1, such as a structural formula or skeletal formula.

Preferably, the first machine learning model 7 has and/or uses one or more artificial neural networks, preferably one or more graph neural networks. Particularly preferably, the artificial or graph neural networks are generative adversarial networks. The use of such networks has proven to be particularly advantageous for generating structures 1.

Generative adversarial networks are in particular described in the article "Generative Adversarial Nets" by Ian J. Goodfellow et al., 2014. This article is available under https://arxiv.org/abs/1406.2661 and is also published in Advances of Neural Information Processing Systems 27 (NIPS 2014).

The first machine learning model 7, in particular the artificial neural network, preferably has a generator 11 and preferably a discriminator 12.

Preferably, the generator 11 is a machine learning model and/or has an artificial neural network, in particular a graph neural network. The generator 11 preferably comprises and/or uses the first training dataset 10.

Preferably, the discriminator 12 is a machine learning model and/or has an artificial neural network, in particular a graph neural network. The discriminator 12 preferably comprises and/or uses the first training dataset 10.

The first training dataset 10 is preferably used for training both the generator 11 and the discriminator 12. In other words, during training, the first training dataset 10 is preferably used as an input for the generator 11 and as an input for the discriminator 12.

Preferably, the generator 11 and the discriminator 12 are separate machine learning models. Preferably, the generator 11 and the discriminator 12 have different or separate machine learning algorithms and/or have or use the same training dataset 10.

The generator 11 and the discriminator 12 preferably form a pair of generative adversarial networks. Thus, the generator 11 and the discriminator 12 preferably contest with each other in a game, in particular in the form of a zero-sum game, where one agent's gain is the other agent's loss.

Particularly preferably, the generator 11 is formed by a generative model G and/or the discriminator 12 is formed by a discriminative model D as described in the above-cited article "Generative Adversarial Nets" by Goodfellow et al.

The first machine learning model 7 is preferably trained using the generator 11 and the discriminator 12. The generator 11 and the discriminator 12 preferably mutually train each other.

The generator 11 preferably generates the structures 1, in particular starting from the molecular and/or empirical formula 6.

For and/or during training, the structures 1 generated by the generator 11 are preferably presented or fed to the discriminator 12. The task of the discriminator 12 during training is to differentiate between structures 1 generated by the generator 11 and structures 1 of real molecules. The structures 1 of real molecules are preferably taken from the first training dataset 10.

In particular, the discriminator 12 is trained to learn the difference between structures 1 generated by the generator 11 and real structures 1. This is in particular done by comparing structures 1 generated by the generator 11 and real structures 1, which are particularly taken from the first training dataset 10, and giving feedback to the discriminator 12 if its decisions are correct.

A real structure 1 is in particular the structure 1 of a molecule that exists in reality, for example the structure 1 of a molecule that has been synthesized or isolated before. Real structures 1 are in particular structures 1 contained in the first training dataset 10.

Preferably, the decisions of the discriminator 12 are, in turn, presented to the generator 11. By this, the generator 11 in particular receives feedback about the generated structures 1 and learns how a real structure 1 "looks". In this way, the generator 11 preferably learns or is trained to generate realistic and/or physically and/or chemically possible structures 1.

The goal of the (mutual) training of the generator 11 and the discriminator 12 is that the generator 11 becomes so good at generating structures 1 that all structures 1 generated by the generator 11 are realistic and/or physically and/or chemically possible. In other words, the generator 11 preferably learns to "fool" the discriminator 12.

When training is completed, it is preferably not possible to differentiate structures 1 generated by the generator 11 from real structures 1 or structures 1 from the first training dataset 10.

The discriminator 12 is preferably used only in the training (phase) and/or not used after the training (phase). The discriminator 12 is preferably not used in the application phase and/or actual structure elucidation and/or used (only) before the application phase.

The first machine learning model 7 preferably generates the structure(s) 1 from the molecular and/or empirical formula 6 in one algorithm iteration, in particular in only or exactly one algorithm iteration.

The first machine learning model 7 and/or generator 11 preferably has and/or uses fully connected layers.

The first machine learning model 7, in particular the generator 11, preferably uses or has a fully connected input graph. The structures 1 generated or to be generated by the first machine learning model 7 are preferably represented by graphs, in particular fully-connected graphs.

Preferably, the first machine learning model 7, in particular the generator 11, generates and/or is trained to generate several and/or different structures 1 from a given or the same molecular and/or empirical formula 6. This is in particular done by using random variables and/or a random noise generator. In other words, the first machine learning model 7, in particular the generator 11, preferably generates and/or is trained to generate structures 1 using a random noise generator and/or random variables. By this, diversity of generated structures 1 can be ensured.

Preferably, the atoms or nuclei of a structure 1 are represented by nodes of a graph and the connections or chemical bonds between the atoms or nuclei are represented by edges of the graph.

It is preferred that to each node in the graph or each nucleus of the structure 1 to be generated, a feature vector is assigned.

The feature vector preferably comprises data identifying the atom or nucleus represented by the respective node. Preferably, this data is the element symbol (e.g., O for oxygen, C for carbon, N for nitrogen, etc.) and/or atomic number (e.g., 8 for oxygen, 6 for carbon, 7 for nitrogen, etc.) of the atom or nucleus represented by the respective node. The atomic number is in particular the number of protons contained in the nucleus or atom. In other words, it is preferred that one element of the feature vector contains the element symbol and/or atomic number represented by the respective node. However, other suitable data for identifying the atom or nucleus represented by the respective node may be used.

Preferably, the feature vector comprises a random variable, in particular in addition to the data identifying the atom or nucleus represented by the respective node. In other words, it is preferred that one element of the feature vector contains a random variable. This preferably ensures randomness of the model.

The random variables of the feature vectors are preferably generated by a random noise generator and/or according to a probability distribution, for example a uniform or Gaussian probability distribution.

Thus, it is particularly preferred that a feature vector is assigned to each node, each node representing one atom or nucleus of a structure 1, the feature vector having at least or exactly two elements, wherein one element contains data identifying the atom or nucleus represented by the respective node, in particular its atomic number and/or element symbol, and one element contains a random variable.

However, the feature vector can also have more than two elements. In particular, the feature vector can comprise further features, such as, in particular chemical, information about the node and/or the molecule, in particular in addition to the data identifying the atom or nucleus represented by the respective node and the element containing a random variable.

For example, the further features, such as, in particular chemical, information about the node and/or the molecule, might be the sum formula of the molecule and/or information about possible neighbors of the atom or nucleus represented by the respective node, about a chemical structure such as ring and/or a certain functional group of which the atom or nucleus represented by the respective node forms a part, about a preferred type of binding and/or preferred binding partners, about the number of valence electrons or the like.

The further information is preferably represented by and/or contained in one or more elements of the feature vector.

The below table shows as an example a graph representation, in particular the feature vectors, of the molecule dicyanoketene, which has the molecular formula C₄N₂O.

| graph node index i | 1 | 2 | 3 | 4 | 5 | 6 | 7 |
|---|---|---|---|---|---|---|---|
| element symbol | O | C | C | C | N | C | N |
| atomic number anum_i | 8 | 6 | 6 | 6 | 7 | 6 | 7 |
| further feature feat_i | feat_1 | feat_2 | feat_3 | feat_4 | feat_5 | feat_6 | feat_7 |
| random variable z_i | z_1 | z_2 | z_3 | z_4 | z_5 | z_6 | z_7 |
| feature vector [anum_i, feat_i, z_i] | [8, feat_1, z_1] | [6, feat_2, z_2] | [6, feat_3, z_3] | [6, feat_4, z_4] | [7, feat_5, z_5] | [6, feat_6, z_6] | [7, feat_7, z_7] |

In particular, the use of the random noise generator and/or random variables in the feature vectors ensures randomness of the model and enables the first machine learning model 7 and/or generator 11 to generate several and/or different structures 1 from one molecular and/or empirical formula 6. Providing further features, in particular chemical, information about the node and/or the molecule, can help in generating realistic structures 1 and can in particular make the training and/or the generation of structures 1 quicker and/or more efficient and/or reliable.

Particularly, from a given molecular and/or empirical formula 6, several and/or different structures 1 are generated by the first machine learning model 7, in particular the generator 11, by applying the machine learning model 7 or the generator 11 to the same molecular and/or empirical formula 6 several times, wherein different random variables, in particular different random variables in the feature vectors, are used in each instance. In particular, the use of different random variables in the feature vectors preferably results in different generated structures 1 although the molecular and/or empirical formula 6 is the same in each instance.

As an alternative to the use of the random noise generator and/or random variables, randomness of the model may also be ensured or achieved in other ways.

For example, instead of using random variables in the feature vector, it is also possible to use feature vectors having only one element containing data identifying the atom or nucleus represented by the respective node and to randomize the edges or connections between the nodes. Thus, in such an approach, the input graph is not fully connected.

### Second machine learning model

According to the invention, generating the predicted spectra 8 from the structures 1 is done by a machine learning model 9 having a residual neural network, also known as a ResNet. In some embodiments of the disclosure that do not form part of the invention, the second machine learning model 9 may have and/or use an artificial neural network other than a residual neural network, preferably a graph neural network, particularly a graph attention network.

A graph attention network is in particular an artificial neural network having a graph attentional layer. Graph attention networks are in particular described in the article "Graph attention networks" by Veličković et al., 2018. This article is available under https://arxiv.org/abs/1710.10903.

A graph attention layer is in particular a layer in which attention coefficients *eᵢⱼ* = ***W**h̅ᵢ, **W**h̅ⱼ* ) are computed, wherein *W* is a learnable weight matrix and ***h*** = {*̅h̅*̅₁, *̅h̅*̅₁, ..., *h̅_{N}*}, ${\vec{h}}_{i} ∈ {ℝ}^{F}$ is the input to the graph attention layer, where *N* is the number of nodes and *F* is the number of features in each node. The attention coefficients *eᵢⱼ* are preferably normalized, in particular using the softmax function. The preferably normalized attention coefficients are used to compute a linear combination of the features corresponding to them, to serve as the final output features for every node of the graph attention layer. In the simplest form, the output ***h'*** = {*h̅'*₁*, h̅'*₁, *... , h̅'_{N}*}, ${\vec{h ′}}_{i} ∈ {ℝ}^{F ′}$ is computed as *h̅'ᵢ* = *σ*( 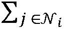 *αᵢⱼ**W** h̅ⱼ*), wherein *σ* is an optional nonlinearity, $W ∈ {ℝ}^{F ′} \times {ℝ}^{F}$ is a learnable weight matrix and *αᵢⱼ* are the normalized attention coefficient, in particular *αᵢⱼ* = softmax(*eᵢⱼ*). Further mathematical details about graph attentional layers can be found in the above-cited article "Graph attention networks" by Veličković et al., 2018, in particular in section 2.1 thereof.

Residual neural networks or ResNets are in particular described in the article "Deep residual learning for image recognition" by He et al., 2015. This article is available under https://arxiv.org/abs/1512.03385 and is also published in Proceedings of the IEEE Conference on Computer Vision and Pattern Recognition (CVPR), 2016, pp. 770-778.

A residual neural network is an artificial neural network having one or more residual blocks. A residual block is a block comprising two layers, wherein x is an input to the residual block and/or the first layer of the residual block, y is an output of the residual block, F(x) is an output of the second layer of the residual block, and wherein the output of the residual block is y = F(x) +x. Thus, the residual block has the input x and the output y = F(x) + x. Thus, the residual block is realized by adding the input x to the output F(x). Further mathematical details of implementing this concept are explained in the above-cited article "Deep residual learning for image recognition" by He et al., 2015.

The second machine learning model has a spectrum generator 13. The spectrum generator 13 has or is formed by the artificial neural network, in particular the graph neural network and/or the residual neural network.

In particular, the second machine learning model 9 and/or spectrum generator 13 has and/or uses a combination of a graph attention network with a residual neural network. Particularly preferably, the second machine learning model 9, in particular the spectrum generator 13, has and/or uses a graph attention network with a residual neural network head.

The second machine learning model 9 and/or spectrum generator 13 preferably has and/or is trained with a training data set 14, hereinafter in particular referred to as second training dataset 14.

In particular, the second machine learning model 9 is trained before using the second machine learning model 9 for actual structure elucidation. In other words, a method according to the present invention preferably involves a training or training phase of the second machine learning model 9 and an application or application phase of the second machine learning model 9.

In the training phase, the second machine learning model 9 preferably learns how predicted spectra 8 are generated, in particular starting from a given structure 1. After training or when the training phase is finished, the second machine learning model 9 is or can be used for generating predicted spectra 8 from structures 1, in particular in order to elucidate the structure 1 of an unknown chemical compound 2 from a measured spectrum 3 of a sample 4.

The application or application phase is preferably the phase after finishing the training phase or, in other words, the phase in which the second machine learning model 9 is used for generating predicted spectra 8 of candidate chemical compounds.

The second machine learning model 9 preferably comprises and/or is formed by the spectrum generator 13 and the second training dataset 14. The spectrum generator 13 preferably is or has an algorithm, in particular a machine learning algorithm.

The spectrum generator 13 preferably generates the spectrum 8 or spectrum features 5, in particular the chemical shifts, in one shot.

The second training dataset 14 preferably comprises structures 1 that are labeled with related spectrum features 5.

The spectrum features 5 are preferably chemical shifts, in particular when the measured spectrum 3 and/or predicted spectrum 8 is an NMR spectrum. Particularly preferably, the spectrum features 5 are ¹H and/or ¹³C chemical shifts. Thus, it is preferred that every ¹H and/or ¹³C atom or nucleus of a given structure 1 contained in the second training dataset 14 is labeled with the related spectrum feature 5 or chemical shift.

The second machine learning model 9, in particular the spectrum generator 13, preferably generates and/or is trained to generate predicted spectra 8 from structures 1. Particularly, the spectra 8 are generated from structures 1 generated by the first machine learning model 7 and/or the structures 1 generated by the first machine learning model 7 are used as input for the second machine learning model 9, in particular the spectrum generator 13.

In particular, for every structure 1, one, in particular exactly or only one, predicted spectrum 8 is computed or generated by the second machine learning model 9 and/or the spectrum generator 13.

The predicted spectra 8 preferably constitute an output of the second machine learning model 9. This is also schematically depicted in Fig. 1.

In the second machine learning model 9 and/or spectrum generator 13, the structures 1 preferably comprise and/or are labeled with chemical information, in particular chemical information about each atom or nucleus of a given structure 1. The chemical information is in particular information about the chemical state of the atom or nucleus, i.e. in particular information about the atom or nucleus itself and its chemical surrounding and/or binding to other atoms or nuclei of the structure 1. The chemical information makes it possible to predict or generate a predicted spectrum 8 and/or spectrum features 5 of the structure 1.

The chemical information about the atom or nucleus preferably comprises information about one of more of the following features: (i) atomic number of the atoms or nuclei and/or other suitable data for identifying the atoms or nuclei; (ii) valence or number of binding partners; (iii) aromaticity, in particular the information if the atom or nucleus is part of an aromatic structure; (iv) hybridization state, such as s, sp, sp², sp³, sp³d or sp³d²; (v) formal charge; (vi) default valence or number of valence electrons; (vii) rings, in particular information if the atom or nucleus is a member of ring and/or information about the size of the ring, e.g. the number of atoms or nuclei forming the ring.

The structures 1 are preferably represented by graphs in the second machine learning model 9 and/or spectrum generator 13. Preferably, the atoms or nuclei of a structure 1 are represented by nodes of a graph.

Preferably, to each node or atom or nucleus, a feature vector is assigned. The feature vector preferably comprises the chemical information of the atom or nucleus represented by the node.

The feature vector is preferably a vector having one or more features. In particular, the feature vector comprises one or more, preferably all, of the features (i) to (vii) listed above.

The features are preferably one-hot encoded. This is conducive to a high computational efficiency and/or a quick computation or generation of the predicted spectra 8.

The second machine learning model 9 and/or spectrum generator 13 preferably generates predicted spectra 8 using a graph attention network. Preferably, for each given structure 1, the second machine learning model 9 and/or spectrum generator 13 generates a node embedding comprising the predicted spectrum 8 of the structure 1 and/or information necessary to compute the predicted spectrum 8 of the structure 1. Thus, in other words, the predicted spectra 8 are preferably represented by and/or encoded in the form of node embeddings. A node embedding is in particular an abstract representation of a predicted spectrum 8 and/or is not human-readable. In other words, while the node embedding preferably contains all information about the predicted spectrum 8 and/or all information needed to display or compute the predicted spectrum 8, the information is not contained in the node embedding in a form that can be directly understood, read or interpreted by a human. In particular, in the node embedding, the predicted spectrum 8 is not contained or represented in the same way as the measured spectrum 3.

According to the invention a residual neural network is used to generate or compute the predicted spectrum 8 from the node embedding. In particular, the residual neural network, outputs the predicted spectrum 8 in the same representation or data type as the measured spectrum 3.

For example, if the measured spectrum 3 is present in the form of a diagram or data table, the generated predicted spectrum 8, in particular the predicted spectrum 8 generated or output by the residual neural network, is also present in the form of a diagram or data table, respectively.

Thus, it is particularly preferred that the second machine learning model 9 and/or spectrum generator 13 has and/or uses a graph attention network and a residual neural network for generating predicted spectra 8 from structures 1, wherein, starting from a structure 1, a node embedding is generated by the graph attention network, the node embedding preferably being a representation or encoding of the predicted spectrum 8, and then the predicted spectrum 8 is generated from the node embedding by the residual neural network.

The second machine learning model 9 or spectrum generator 13 is preferably able to predict the (different) spectrum features 5, in particular chemical shifts, of diastereotopic protons.

In particular, for each spectrum feature 5, in particular chemical shift of the predicted spectrum 8, an expected or mean value and a corresponding measure of dispersion, in particular a standard deviation, are calculated, in particular by the second machine learning model 9 and/or spectrum generator 13. The expected or mean value is preferably the position of the spectrum feature 5 in the predicted spectrum 8. For example, the expected or mean value is the position of the chemical shift, or in other words the "ppm-value" of the chemical shift.

It has in particular turned out that this procedure of calculating both an expected mean value and a corresponding measure of dispersion leads to the ability of the second machine learning model 9 to predict the (different) spectrum features 5, in particular chemical shifts, of diastereotopic protons. Namely, it has turned out that the prediction of the mean values or positions of the spectrum features 5 or chemical shifts is so accurate that normally, the corresponding measure of dispersion or standard deviation is zero. In the case of diastereotopic protons, however, the calculated standard deviations turn out to be larger than zero.

The spectrum features 5 or chemical shifts of diastereotopic protons are preferably calculated by adding and subtracting the corresponding measure of dispersion or standard deviation to the expected or mean value. Preferably, the spectrum feature 5 or chemical shift of one of the diastereotopic protons is the sum of the expected or mean value and the corresponding measure of dispersion and the spectrum feature 5 or chemical shift of the other of the diastereotopic protons is the difference of the expected or mean value and the corresponding measure of dispersion.

For example, when the expected or mean value of two diastereotopic protons is 4.42 ppm and the corresponding measure of dispersion or standard deviation is 0.18 ppm, the spectrum feature 5 or chemical shift of one of the diastereotopic protons is calculated to be 4.60 ppm (= 4.42 ppm + 0.18 ppm) and the calculated position of the second diastereotopic protons is 4.24 ppm (= 4.42 ppm - 0.18 ppm).

Individual aspects in features of the present invention may be implemented independently from one another, but also in any desired combination and/or order.

### List of references signs:

- 1: structure
- 2: unknown chemical compound
- 3: measured spectrum
- 4: sample
- 5: spectrum feature
- 6: molecular and/or empirical formula
- 7: (first) machine learning model
- 8: predicted spectrum
- 9: (second) machine learning model
- 10: (first) training dataset
- 11: generator
- 12: discriminator
- 13: spectrum generator
- 14: (second) training dataset

## Claims

1. Computer-implemented method for structure elucidation of the structure (1) of an unknown chemical compound (2) from a measured spectrum (3) of a sample (4), wherein structures (1) of candidate chemical compounds are generated and predicted spectra (8) are generated from the generated structures (1),
wherein the predicted spectra (8) are compared with the measured spectrum (3), and
wherein one of the predicted spectra (8) is selected and the structure (1) corresponding to the selected predicted spectrum (8) is determined as the structure (1) of the unknown chemical compound (2), and
wherein
a machine learning model (9) generates the predicted spectra (8) from the generated structures (1), the machine learning model (9) having a residual neural network.

2. Method according to claim 1, wherein said machine learning model (9) is trained with a training dataset (14), the training dataset (14) preferably containing structures (1) labeled with related spectrum features (5), preferably chemical shifts, in particular ¹H and/or ¹³C chemical shifts.

3. Method according to claim 1 or 2, wherein
a) a first machine learning model (7) generates the structures (1) of candidate chemical compounds and the aforementioned machine learning model, hereinafter referred to as second machine learning model (9), generates the predicted spectra (8) from the structures (1) generated by the first machine learning model (7), and/or
b) a first machine learning model (7) generates the structures (1) of candidate chemical compounds, wherein the first machine learning model (7) is trained for generating realistic structures (1) from a molecular and/or empirical formula (6).

4. Method according to claim 3, wherein the first machine learning model (7) has one or more artificial neural networks, preferably graph neural networks, in particular a pair of generative adversarial networks.

5. Method according to claim 3 or 4, wherein the first machine learning model (7) is trained with a first training dataset (10), preferably for the generation of realistic structures (1), in particular from molecular and/or empirical formulas (6), the first training dataset (10) preferably comprising structures (1) of a plurality of real molecules.

6. Method according to one of claims 3 to 5, wherein the first machine learning model (7) is trained using a generator (11) and a discriminator (12), preferably wherein the generator (11) and the discriminator (12) mutually train each other and/or wherein the discriminator (12) is used only in the training and/or not used in actual structure elucidation and/or in an application phase.

7. Method according to one of claims 3 to 6, wherein the first machine learning model (7), in particular the generator (11), generates and/or is trained to generate several structures (1) from a given molecular and/or empirical formula (6), in particular using random variables and/or a random noise generator.

8. Method according to claim 6 or 7, wherein the discriminator (12) is trained to differentiate between real structures (1), in particular from the first training data set (10), and structures (1) generated by the generator (11).

9. Method according to one of the preceding claims, wherein an expected or mean value and a corresponding measure of dispersion, in particular a standard deviation, are calculated for each spectrum feature (5), in particular chemical shift, of the predicted spectrum (8).

10. Method according to one of the preceding claims, wherein the measured spectrum (3) is an NMR spectrum and/or wherein a spectrum, in particular an NMR spectrum, of the sample (4) is measured.

11. Method according to one of the preceding claims, wherein the molecular and/or empirical formula (6) is determined by measuring a mass spectrum of the sample (4).

12. Data processing apparatus comprising means for carrying out the method of one of the preceding claims.

13. Computer program product comprising instructions which, when the program is executed by a computer, cause the computer to carry out the method of one of claims 1 to 11.

14. Computer-readable storage medium comprising instructions which, when executed by a computer, cause the computer to carry out the method of one of claims 1 to 11.

## Patentansprüche

1. Computerimplementiertes Verfahren zur Strukturaufklärung der Struktur (1) einer unbekannten chemischen Verbindung (2) aus einem gemessenen Spektrum (3) einer Probe (4),
wobei Strukturen (1) von chemischen Verbindungskandidaten erzeugt werden und aus den erzeugten Strukturen (1) vorhergesagte Spektren (8) erzeugt werden,
wobei die vorhergesagten Spektren (8) mit dem gemessenen Spektrum (3) verglichen werden, und
wobei eines der vorhergesagten Spektren (8) ausgewählt wird und die dem ausgewählten vorhergesagten Spektrum (8) entsprechende Struktur (1) als die Struktur (1) der unbekannten chemischen Verbindung (2) bestimmt wird, und
wobei
ein Machine-Learning-Modell (9) die vorhergesagten Spektren (8) aus den erzeugten Strukturen (1) erzeugt, wobei das Machine-Learning-Modell (9) ein neuronales Residualnetzwerk aufweist.

2. Verfahren nach Anspruch 1, wobei das Machine-Learning-Modell (9) mit einem Trainingsdatensatz (14) trainiert wird, wobei der Trainingsdatensatz (14) vorzugsweise Strukturen (1) enthält, die mit zugehörigen Merkmalen des Spektrums (5), vorzugsweise chemischen Verschiebungen, insbesondere chemischen ¹H- und/oder ¹³C-Verschiebungen, gekennzeichnet sind.

3. Verfahren nach Anspruch 1 oder 2, wobei
a) ein erstes Machine-Learning-Modell (7) die Strukturen (1) von chemischen Verbindungskandidaten erzeugt und das vorgenannte Machine-Learning-Modell, im Folgenden als zweites Machine-Learning-Modell (9) bezeichnet, die vorhergesagten Spektren (8) aus den durch das erste Machine-Learning-Modell (7) erzeugten Strukturen (1) erzeugt, und/oder
b) ein erstes Machine-Learning-Modell (7) die Strukturen (1) von chemischen Verbindungskandidaten erzeugt, wobei das erste Machine-Learning-Modell (7) zur Erzeugung realistischer Strukturen (1) aus einer molekularen und/oder empirischen Formel (6) trainiert wird.

4. Verfahren nach Anspruch 3, wobei das erste Machine-Learning-Modell (7) ein oder mehrere künstliche neuronale Netzwerke, vorzugsweise neuronale Graphennetzwerke, insbesondere ein Paar von erzeugenden gegnerischen Netzwerken, aufweist.

5. Verfahren nach Anspruch 3 oder 4, wobei das erste Machine-Learning-Modell (7) mit einem ersten Trainingsdatensatz (10), vorzugsweise zur Erzeugung realistischer Strukturen (1), insbesondere aus molekularen und/oder empirischen Formeln (6), trainiert wird, wobei der erste Trainingsdatensatz (10) vorzugsweise Strukturen (1) einer Vielzahl von realen Molekülen aufweist.

6. Verfahren nach einem der Ansprüche 3 bis 5, wobei das erste Machine-Learning-Modell (7) unter Verwendung eines Generators (11) und eines Diskriminators (12) trainiert wird, vorzugsweise wobei der Generator (11) und der Diskriminator (12) sich gegenseitig trainieren und/oder wobei der Diskriminator (12) nur beim Training verwendet wird und/oder nicht bei der tatsächlichen Strukturaufklärung und/oder in einer Anwendungsphase verwendet wird.

7. Verfahren nach einem der Ansprüche 3 bis 6, wobei das erste Machine-Learning-Modell (7), insbesondere der Generator (11), mehrere Strukturen (1) aus einer gegebenen molekularen und/oder empirischen Formel (6) erzeugt und/oder zur Erzeugung dieser trainiert wird, insbesondere unter Verwendung von Zufallsvariablen und/oder eines Generators für zufälliges Rauschen.

8. Verfahren nach Anspruch 6 oder 7, wobei der Diskriminator (12) trainiert wird, um zwischen realen Strukturen (1), insbesondere aus dem ersten Trainingsdatensatz (10), und durch den Generator (11) erzeugten Strukturen (1) zu unterscheiden.

9. Verfahren nach einem der voranstehenden Ansprüche, wobei für jedes Merkmal des Spektrums (5), insbesondere jede chemische Verschiebung, des vorhergesagten Spektrums (8) ein Erwartungswert oder Mittelwert und ein entsprechendes Maß der Streuung, insbesondere eine Standardabweichung, berechnet werden.

10. Verfahren nach einem der voranstehenden Ansprüche, wobei das gemessene Spektrum (3) ein NMR-Spektrum ist und/oder wobei ein Spektrum, insbesondere ein NMR-Spektrum, der Probe (4) gemessen wird.

11. Verfahren nach einem der voranstehenden Ansprüche, wobei die molekulare und/oder empirische Formel (6) durch Messung eines Massenspektrums der Probe (4) bestimmt wird.

12. Datenverarbeitungsvorrichtung, umfassend Mittel zur Durchführung des Verfahrens nach einem der voranstehenden Ansprüche.

13. Computerprogrammprodukt, umfassend Anweisungen, die, wenn das Programm von einem Computer ausgeführt wird, den Computer zur Durchführung des Verfahrens nach einem der Ansprüche 1 bis 11 veranlassen.

14. Computerlesbares Speichermedium, umfassend Anweisungen, die, wenn sie von einem Computer ausgeführt werden, den Computer zur Durchführung des Verfahrens nach einem der Ansprüche 1 bis 11 veranlassen.

## Revendications

1. Procédé mis en œuvre par ordinateur pour l'élucidation de structure de la structure (1) d'un composé chimique inconnu (2) à partir d'un spectre mesuré (3) d'un échantillon (4),
dans lequel des structures (1) de composés chimiques candidats sont générées et des spectres prédits (8) sont générés à partir des structures générées (1),
dans lequel les spectres prédits (8) sont comparés au spectre mesuré (3), et
dans lequel l'un des spectres prédits (8) est sélectionné et la structure (1) correspondant au spectre prédit sélectionné (8) est déterminée comme étant la structure (1) du composé chimique inconnu (2), et
dans lequel
un modèle d'apprentissage automatique (9) génère les spectres prédits (8) à partir des structures générées (1), le modèle d'apprentissage automatique (9) ayant un réseau neuronal résiduel.

2. Procédé selon la revendication 1, dans lequel ledit modèle d'apprentissage automatique (9) est entraîné avec un ensemble de données d'entraînement (14), l'ensemble de données d'entraînement (14) contenant de préférence des structures (1) marquées avec des caractéristiques de spectre associées (5), de préférence des déplacements chimiques, en particulier des déplacements chimiques ¹H et/ou ¹³C.

3. Procédé selon la revendication 1 ou 2, dans lequel
a) un premier modèle d'apprentissage automatique (7) génère les structures (1) de composés chimiques candidats et le modèle d'apprentissage automatique susmentionné, appelé ci-après second modèle d'apprentissage automatique (9), génère les spectres prédits (8) à partir des structures (1) générées par le premier modèle d'apprentissage automatique (7), et/ou
b) un premier modèle d'apprentissage automatique (7) génère les structures (1) de composés chimiques candidats, dans lequel le premier modèle d'apprentissage automatique (7) est entraîné pour générer des structures réalistes (1) à partir d'une formule moléculaire et/ou empirique (6).

4. Procédé selon la revendication 3, dans lequel le premier modèle d'apprentissage automatique (7) a un ou plusieurs réseaux neuronaux artificiels, de préférence des réseaux neuronaux graphiques, en particulier une paire de réseaux antagonistes génératifs.

5. Procédé selon la revendication 3 ou 4, dans lequel le premier modèle d'apprentissage automatique (7) est entraîné avec un premier ensemble de données d'entraînement (10), de préférence pour la génération de structures réalistes (1), en particulier à partir de formules moléculaires et/ou empiriques (6), le premier ensemble de données d'entraînement (10) comprenant de préférence des structures (1) d'une pluralité de molécules réelles.

6. Procédé selon l'une des revendications 3 à 5, dans lequel le premier modèle d'apprentissage automatique (7) est entraîné en utilisant un générateur (11) et un discriminateur (12), de préférence dans lequel le générateur (11) et le discriminateur (12) s'entraînent mutuellement et/ou dans lequel le discriminateur (12) est utilisé uniquement dans l'entraînement et/ou n'est pas utilisé dans l'élucidation de structure réelle et/ou dans une phase d'application.

7. Procédé selon l'une des revendications 3 à 6, dans lequel le premier modèle d'apprentissage automatique (7), en particulier le générateur (11), génère et/ou est entraîné pour générer plusieurs structures (1) à partir d'une formule moléculaire et/ou empirique donnée (6), en particulier en utilisant des variables aléatoires et/ou un générateur de bruit aléatoire.

8. Procédé selon la revendication 6 ou 7, dans lequel le discriminateur (12) est entraîné pour différencier des structures réelles (1), en particulier à partir du premier ensemble de données d'entraînement (10), et des structures (1) générées par le générateur (11).

9. Procédé selon l'une des revendications précédentes, dans lequel une valeur attendue ou moyenne et une mesure correspondante de dispersion, en particulier un écart-type, sont calculées pour chaque caractéristique de spectre (5), en particulier un déplacement chimique, du spectre prédit (8).

10. Procédé selon l'une des revendications précédentes, dans lequel le spectre mesuré (3) est un spectre RMN et/ou dans lequel un spectre, en particulier un spectre RMN, de l'échantillon (4) est mesuré.

11. Procédé selon l'une des revendications précédentes, dans lequel la formule moléculaire et/ou empirique (6) est déterminée en mesurant un spectre de masse de l'échantillon (4).

12. Appareil de traitement de données comprenant des moyens pour mettre en œuvre le procédé selon l'une des revendications précédentes.

13. Produit de programme d'ordinateurcomprenant des instructions qui, lorsque le programme est exécuté par un ordinateur, conduisent celui-ci à mettre en œuvre le procédé selon l'une des revendications 1 à 11.

14. Support d'enregistrement lisible par ordinateur comprenant des instructions qui, lorsqu'elles sont exécutées par un ordinateur, conduisent celui-ci à mettre en œuvre le procédé selon l'une des revendications 1 à 11.
